# EUROPEAN PATENT SPECIFICATION

(11) **EP 1 320 325 B1**
(45) Date of publication and mention of the grant of the patent: **24.09.2008**
(21) Application number: 01974654.4
(22) Date of filing: 25.09.2001
(51) Int. Cl.: A61B 6/00, A61B 10/00

(54) **APPARATUS FOR PROSTATE CANCER DIAGNOSE**
GERÄT FÜR PROSTATEKREBSDIAGNOSE
DISPOSITIF POUR DIAGNOSTIQUER LE CANCER DE LA PROSTATE

(30) Priority: 28.09.2000 IL 13875600
(43) Date of publication of application: 25.06.2003
(73) Proprietor: YEDA RESEARCH AND DEVELOPMENT CO., LTD., 76100 Rehovot (IL); THE STATE of ISRAEL Atomic Energy Commission Soreq Nuclear Research Center, Yavne 81800 (IL)
(72) Inventor: BRESKIN, Amos, 74051 Nes Ziona (IL); CHECHIK, Rachel, 76868 Moshav Beit Haran (IL); VARTSKY, David, 76308 Rehovot (IL)
(74) Representative: Schmitz, Jean-Marie
(86) International application number: PCT/IL2001/000902
(87) International publication number: WO 2002/026130

(56) References cited:
- V.YE.ZAICHICK ET AL: "Zinc in the Human Prostate Gland: Normal, Hyperplastic and Cancerous" INTERNATIONAL UROLOGY AND NEPHROLOGY, vol. 29, no. 5, 30 May 1997 (1997-05-30), pages 565-574, XP008001184 Russia
- DATABASE WPI Derwent Publications Ltd., London, GB; AN 1981-39811D XP002192460 "DIFFERENTIAL DIAGNOSE OF TUMOURS OF THE PROSTATE BY MEASURING THE CONCENTRATE OF ZINC IN TUMOROUS TISSUE BY X_RAY FLUORESCENT ANALYSIS" & SU 764 660 A (AM SCI MED RADIOLOG), 23 September 1980 (1980-09-23)

## Description

The present invention is directed to an apparatus for detection of chemical elements in the prostate using the X-ray fluorescence method and, particularly, to an apparatus for detection and staging of prostate cancer by *in vivo* determination and mapping of Zn in the prostate.

In order to describe the state of the art in the field of the invention, mention of the following references will be made:
1. Catalona, W.J., Clinical utility of measurements of free and total prostate-specific antigen (PSA): A review, Prostate 7:64, (1996).
2. Catalona, W.J., Paitin, A.W., Finlay, J.A., eChan, D.W., Rittenhouse, HJG., Wolfert, R.L., and Woodrum, D.L., Use of percentage of free prostate specific antigen to identify men at high risk of prostate cancer when PSA levels are 2.51 to 4 ng/ml and digital examination is not suspicious for prostate cancer: An alternative model, Urology, 54:220-224, (1999).
3. Cotran RS., Kumar V. and Collins T., Robbins Pathologic Basis of Disease, W.B. Sounders Co., Sixth Edition, 1029, (1999).
4. Gyorkey, F., Min K. W., Huff, J.A. and Gyorkey, P., Zinc and magnesium in human prostate gland: normal hyperplastic and neoplastic, Cancer, 27:1348, (1967).
5. Lahtonen, R, Zinc and cadmium concentrations in whole tissue and separated epithelium and stroma from human benign prostatic hypertrophic glands. Prostate, 6:177, (1985).
6. Gonic, P., Oberleas D., Knechtges T. and Prasad, A.S., Atomic absorption determination of zinc in the prostate. Invest. Urol., 6:345, (1969).
7. Dhar, N.K., Goel, T.C., Dube, P.C., Chowdury, A.R. and Kar, A.B., Distribution and concentration of zinc in the subcellular fractions of benign hyperplastic and malignant neoplastic human prostate, Exp. Mol. Pathol., 19:139, (1973).
8. Habib, F.K., Mason, M.K., Smith, P.H., and Stitch, S.R., Cancer of the prostate: early diagnosis by zinc and hormone analysis, Br. J. Cancer 39:700, (1979).
9. Ogunlewe, J.O. and Osegbe, DN., Zinc and cadmium concentrations in indigenous blacks with normal, hypertrophic and malignant prostate, Cancer, 63:1388, (1989).
10. Feustel, A., Wennrich, R., Steiniger, D. and Klauss, P., Zinc and cadmium concentration in prostatic carcinoma of different histological grading in comparison to normal prostate tissue and adenofibromyomatosis (BPH), Urol. Res. 10:301, 1982).
11. Zaichick, V.Y, Sviridova, T.V and Zaichick, S.V, Zinc in the human prostate gland, normal, hyperplastic and cancerous, Int. Urol. Nephrol., 29:687-694, (1997).

Carcinoma of the prostate is the most common form of cancer in men. The methods commonly used today for detection of prostate cancer are digital rectal examination (DRE), transrectal ultrasound (TRUS) and prostate-specific-antigen (PSA) determination. There are large uncertainties in each one of them. Therefore, the diagnosis is often based on a combination of these examinations.

PSA testing is the most common assay used in diagnosis of prostate cancer and particularly in screening. In normal men, only minute amounts of PSA circulate in the serum. Elevated PSA levels in blood occur in association with localized as well as advanced prostate cancer. In most laboratories a serum level of 4 ng/ml is used as a cut-off point between normal and abnormal. However, elevations in PSA occur not only in cancer cases but also in some non-neoplastic conditions, such as nodular hyperplasia and prostatitis. There is a considerable overlap in levels of serum PSA between that found in such conditions and that found in prostate cancer patients. For instance, 25 to 30% of men with nodular hyperplasia and 80% with histologically documented cancer have PSA serum level greater than 4 ng/ml.

In view of this large overlap, several refinements in the estimation and interpretation of PSA values have been proposed, such as PSA density (ratio of PSA level to the volume of the prostate gland), PSA velocity (rate of change in the PSA level over time), age-specific reference values and the ratio of free and total PSA in the serum (percent free free PSA; %FPSA). Although it appears that %FPSA is valuable in discriminating between benign and malignant disease for cases where the total PSA is in the "*gray zone*" of 4 to 10 ng/ml, until the value of the above mentioned refinements is better established, serum PSA by itself cannot be used for detection of early cancer and must be combined with other diagnostic indicators.

In addition to the above-mentioned deficiencies, the existing methods do not provide sufficient information about the stage of the disease, namely the tumor dimension and the level of cancer proliferation. Moreover, when cancer is suspected a biopsy procedure is usually performed. The lack of precise information as to the tumor localization renders the biopsy procedure inefficient.

It is well established that a normal human prostate gland contains high levels of zinc (Zn). Although reported values vary considerably, whole prostate preparations contain Zn concentrations of about 150 µg/g wet weight, which is about 2-5 times greater than Zn content of most other tissues. Zinc is not uniformly distributed throughout the prostate and, as demonstrated by Gyorkey *et al*., 1967, the highest Zn content (211 µ/g wet weight) is found in the lateral lobe of the peripheral zone. Numerous *in vitro* studies (Gyorkey *et al*. 1967, Lahtonen, 1985, Gonic *et al*., 1969, Dhar *et al*., 1973; Habib *et al*., 1979; Ogunlewe and Osegbe, 1989; Feustel *et al*., 1982; and Zaichick *et al*., 1997) indicate that Zn concentration in the prostate is substantially lower in cancerous tissue compared to benign prostate hyperplasia (BPH) and normal prostate tissue. Zaichick *et al*., (1997) reported dry weight Zn concentrations of 1018 ± 124, 1142 ± 77 and 146 ± 10 µg/g for normal, BPH and cancerous prostate, respectively. In addition, they found that the decrease in Zn levels in cancer starts at very early stages of the disease and there is a lack of Zn level dependence on the stages of the disease and on histological cancer grading. Zinc levels are modified only in the cancerous tissue. Tissue not involved in the tumor process remain unaltered and zinc levels in visually and morphologically intact tissues are at normal levels.

It is important to note that the Zn levels in prostate cancer approach the typical levels normally associated with non-prostate tissue, which would indicate that the malignant prostate epithelial cells have lost the ability to accumulate zinc. Based on this finding, Habib *et al*., 1979, suggested that the decrease in zinc was an early step in malignancy and could be used for early diagnosis of prostate cancer. It has been suggested that decreased zinc accumulation occurs in cell population prior to their histopathological identification as malignant cells and that this represents biochemical changes early in the malignant process, possibly as a premalignant stage (Cotran *et al*., 1999).

In view of the above findings and due to the fact that in most of the cases, carcinoma of the prostate originates in the peripheral zone of the gland, classically in a posterior location accessible through the rectum, it seems plausible that *in vivo* measurement of Zn in the prostate is feasible and could aid in more reliable differentiation between cancerous tissue and that of benign prostate hyperplasia and normal tissue. Thus, it may reduce the rate of false-negative diagnosis and therefore the required number of biopsies, with the important consequence of cost reduction in healthcare. Moreover, it may reduce the rate of false-negative diagnosis, thus minimizing the mortality from undetected prostate cancer.

The important observation by Zaichick *et al*, 1997, that zinc concentration is modified only in the affected tissue *may* permit cancer staging by mapping the distribution of Zn within the prostate. This aspect is most important at the preoperative stage. It is the basis for decisions regarding the recommended treatment, whether partial- or radical-surgical or other type of treatment.

In accordance with the invention an apparatus is provided that permit effective detection of Zn and other elements in human prostate tissue in vivo. These are useful for the diagnosis and staging of prostate cancer.

The apparatus of the present invention is defined in claim 1.

In accordance with the present invention, the X-ray fluorescence (XRF) technique is used in detection of Zn and other elements in *vivo* in the human prostate. In XRF, the analyzed tissue is exposed to a low radiation dose of X-rays o low energy gamma rays from an X-ray tube or an isotopic radioactive source. This radiation causes the excitation of the atoms present in the tissue, which in turn decay by emission of characteristic fluorescent X-rays. The characteristic X-rays emitted from the irradiated area are detected and counted by a detector with high energy-resolution. The intensity of these X-rays is directly proportional to the concentration of the elements inside the tissue. In case of Zn, the characteristic X-ray energies are 8.6 and 9.6 keV.

In the following the term "*determine*" or "*determination*" will, at times, be used to denote one or both of qualitative determination, namely detecting the presence of a certain element in the prostate tissue, or quantitative determination, namely determination of the amount or level of an element in the tissue.

The present invention relates to an apparatus for *in vivo* detection of chemical elements in the prostate.

In one preferred embodiment, the chemical element to be detected is Zn.

In other embodiments, the element may be any other element normally present in the prostate glad tissue such as Fe, Ca, or Br. These elements may be determined simultaneously with Zn by the XRF technique and can serve for normalization purposes.

In another embodiment, the elements to be determined are elements that are introduced into the prostate for the purpose of a specific medical procedure, for example, for therapeutic purposes. These can, for example, be palladium (Pd), in the form of Pd-porphyrin compounds used in photodynamic therapy (PDT).

The radiation source is a source that generates radiation of an energy such that it can excite elements to emit a fluorescent X-ray radiation. The radiation source may be selected from a radioactive source, an X-ray tube, a synchrotron light source, an X-ray beam guide connected to an external X-ray source, a miniature plasma X-ray generator and others. A radioactive source may for example be ¹⁰⁹Cd. An X-ray tube may, for example, be a miniature X-ray tube such as, for example, plasma X-ray source. When used for tissue mapping, said radiation source is typically a scanning source.

The radiation detector may be selected from a high energy-resolution solid state detector such as detectors based on Silicon (Si), Silicon-Lithium-drifted (Si(Li)), Mercury Iodide (HgI₂) or Cadmium-Zinc Telluride (CdZnTe), which can be cooled by a small thermoelectric device, or any other detector sensitive to X-rays, or an array of such detectors. When used for mapping, said radiation detector is typically a scanning detector or a position-sensitive detector. In case of a scanning detector, its scan is essentially synchronized with the scan of the radiation source.

The collimation system that is generally known *per se*, should prevent the said radiation detector from directly receiving any radiation emitted from the said radiation source, and to receive only radiation emitted from the elements in the prostate tissue under investigation. The collimation system should be constructed of materials whose characteristic X-rays do not interfere with the determination of the tissue elements particularly Zn.

It is known that in most cases (70-80%), carcinoma of the prostate originates in the peripheral zone of the posterior lobe, which may be diagnosed by access through the rectum. For the purpose of diagnosing other (central) parts of the prostate access is preferably through the urethra. Thus, the probe according to the invention is adapted for at least one of transrectal and transurethral examination.

In one embodiment, the transrectal probe may be combined with or may include a transrectal ultrasound probe (TRUS).

In another embodiment, the transrectal or transurethral probe includes a biopsy device.

In still another embodiment, the transrectal or transurethral probe includes a device for injection of a drug, in which case the probe both localizes the cancerous areas of the prostate and injects into it the drug that may be a therapeutic drug or a drug for tissue imaging, e.g. a contrast agent.

In an additional embodiment, the probe includes a device for illumination of an identified cancerous site with a light beam. The light beam is typically a selected therapeutic wavelength for photodynamic therapy.

In another embodiment the probe can be used as a detector for radioactive substances introduced into the prostate for diagnostic purposes, for example, radioactive I or Zn. In such a mode of operation, the exciting radiation emanating from the probe is typically turned off. This may be done through a peripheral device or through an ON/OFF switch included within the probe.

In a further embodiment, the probe of the invention may be used as an intraoperative probe.

In another aspect, the present invention relates to an apparatus for *in vivo* detection of chemical elements in the prostate by X-ray fluorescence comprising a probe of the invention, a signal amplification unit and a signal recording, processing and displaying system.

With the apparatus of the present invention a method for *in vivo* detection of one or more chemical elements in the prostate can be performed, the method comprising:
(a) introducing a probe of the invention transrectally in close proximity to the posterior lobe of the prostate of said individual or transurethrally in close proximity to the lobus medius of the prostate of said individual; and
(b) irradiating the prostate by the radiation source while simultaneously measuring emitted characteristic fluorescent X-ray radiation of the elements present in the prostate.

For determination of the level of a measured element in the prostate, the intensity of the fluorescent X-ray radiation may be measured, such intensity being in correlation with said level. Thus, for determining said level the method further comprises:
(c) determining the intensity of the characteristic fluorescent X-ray radiation; and
(d) calculating the level of each of said elements in the prostate based on the measured intensity of said radiation.

The level of an element may be determined by absolute fluorescent radiation levels, although this may introduce inaccuracy since the absolute measured fluorescent radiation may depend on a number of factors such as probe position, e.g. distance of the probe from the tissue, probe sensitivity and others. Thus for a quantitative determination, a normalizing measurement of a reference element whose level is relatively constant may be made and the equal determination of the level may then be made in comparison to the reference element, for example, a reference element is Fe, Ca or Br.

With the apparatus of the present invention a method for diagnosing prostate cancer in an individual can be performed, the method comprising:
(a) introducing a probe of the invention transrectally in close proximity to the posterior lobe of the prostate of said individual or transurethrally in close proximity to the lobus medius of the prostate of said individual;
(b) irradiating the prostate by the radiation source while simultaneously measuring emitted characteristic fluorescent X-ray radiation of Zn present in the prostate;
(c) determining the intensity of the fluorescent X-ray radiation; and
(d) calculating the level of Zn in the prostate based on the measured intensity of said radiation, a level of Zn below a threshold level indicating a high probability of a cancerous pathological condition of the prostate.

The method carried out with the apparatus of the present invention may also involve the measurement of the level of radioactive elements introduced to the prostate by either systemic or local administration (into the prostate or proximal thereto). This may be useful in staging of the disease.

In one preferred embodiment, the introduced radioactive element is ¹²⁵I and a modification in the ¹²⁵I concentration levels in the prostate indicates a cancerous pathological condition of the prostate.

According to the present invention, experiments were carried out demonstrating that zinc can be accurately measured in the prostate by the XRF method without interference from other elements. It has been further found in accordance with the present invention that the optimal energy of the incident radiation source for detection of Zn is in the range of 15-20 keV.

The measurement is performed by introducing the probe through the rectum in close proximity to the peripheral zone of the prostate, or through the urethra, in proximity to the central region of the prostate gland. Prostate tissue is irradiated by the incident source radiation and the characteristic element X-rays due to said element, e.g. Zn, present in the tissue are emitted from the tissue and are measured by the radiation detector. The intensity of these X-rays is proportional to the concentration of said element, e.g. Zn, in the prostrate tissue.

By scanning the prostate with the probe it is possible to obtain the distribution of Zn in the region under examination and thus locate regions of varying Zn concentrations, a procedure which can have an importance for staging the prostate cancer and then choosing the best therapeutic approach and procedures.

A combination of the proposed method with other mapping methods such as TRUS provides an improved accuracy for a variety of medical purposes such as for biopsy procedures.

In the following some specific embodiments of the invention will be described with reference to the annexed drawings in which:
**Fig. 1** depicts a diagrammatic illustration of a transrectal or transurethral *in vivo* elemental (e.g. Zn) probe according to the invention.
**Fig. 2** depicts a schematic view of a system used for *in vitro* determination of Zn in prostate phantom.
**Fig. 3** is an XRF spectrum obtained from a prostate phantom vial containing 1000 µg/g of Zn in aqueous solution, with the system of Fig. 2.
**Fig. 4** is an XRF spectrum (logarithmic vertical scale) obtained from a prostate sample diagnosed for adenoma.
**Fig. 5** is an XRF spectrum (logarithmic vertical scale) obtained from a prostate sample diagnosed for cancer.
**Fig. 6** is the illustration of the transrectal prostate examination procedure using the probe of the invention.

The invention relates to an apparatus for *in vivo* determination of elements, particularly Zn, in the human prostate.

A method carried out with the apparatus of the present invention is useful as diagnostic tool for prostate cancer, particularly early prostate cancer and for cancer staging.

In one preferred embodiment, the instrument used for diagnosis consists of an X-ray fluorescence (XRF) system including a rectal probe, which is brought in close proximity to the backside (posterior) region of the prostate, known as the region where most cases of cancer begin their development. The probe contains a small X-ray source, of a moderate intensity, that irradiates the prostate and a detector that records and at times also maps the characteristic X-rays re-emitted from the prostate. By this technique, it is possible to evaluate the Zn concentration in the prostate with good accuracy and to obtain the required information about its distribution.

Since Zn concentrations in the prostate are about 5 times lower in cancerous tissue compared to normal and benign prostate hyperplasia (BPH), an accurate *in vivo* mapping of Zn concentration will have a significant impact on the diagnosis reliability and on preoperative staging. In addition, it is expected that mapping the Zn content in the tissue will provide a clear outline of the cancerous region, which may aid prostate cancer therapy.

X-ray fluorescence (XRF) is an analytical method widely used for analysis of trace elements in various matrices. Biological samples such as tissues can be analyzed intact by XFR without sample processing. In XFR, the analyzed tissue may be exposed to a low radiation dose of X-rays or low energy gamma rays from an X-ray tube or an isotopic radioactive source. This radiation causes the excitation of the atoms present in the tissue, which in turn decay by emission of characteristic fluorescent X-rays. The characteristic X-rays emitted from the sample are detected and counted by a high energy-resolution detector. The intensity of these X-rays is directly proportional to the concentration of the elements inside the tissue. In the case of Zn, the characteristic fluorescent X-ray energies are 8.6 and 9.6 keV. The sensitivity of the XFR method is dependent on the element in question and on the experimental conditions. The limits of detection are usually below 1 µg/g, e.g. 1 part per million.

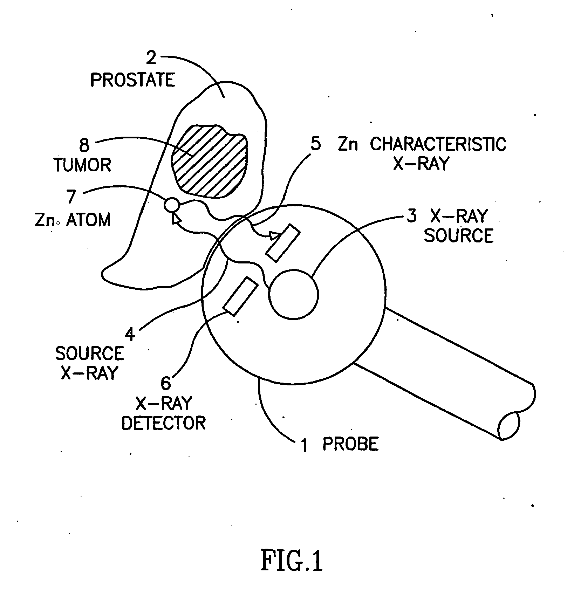

The method carried out with the apparatus of the invention is best explained with reference to Fig. 1. As shown, a probe **1** is placed in a close proximity to the prostate **2,** which may contain a tumor **8.** A radiation source **3** emits X-rays **4** of a desired energy and flux so as to impinge on the prostate tissue. This radiation causes the excitation of Zn atoms **7** present in the tissue, which in turn decay by emission of characteristic fluorescence X-rays **5.** These characteristic X-rays emitted from the tissue are detected and counted by a high energy-resolution detector 6 electrically linked to a recorder outside the probe.

Source **3** may, for example, be a radioactive source, an X-ray tube, a synchrotron light source, an X-ray beam guide connected to an external X-ray source or miniature plasma X-ray generator. The energy of the incident exciting photons is dictated by the energy behavior of the cross-section for the excitation of a given element and by the background produced by scattering of the incident radiation on the large mass of surrounding tissue. In order to reduce this background, the energy of the incident radiation should be well above the characteristic Zn X-rays (8.6 and 9.6 keV). On the other hand, the cross-section for the excitation of Zn decreases for energies above 9.66 keV (the K-edge energy of Zn).

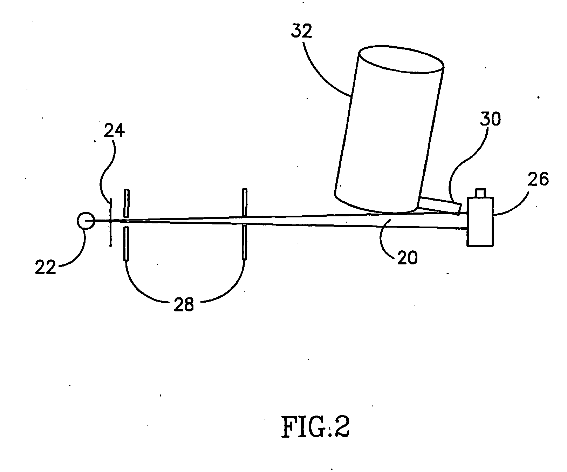

The determination of the optimal energy of the incident radiation and a feasibility of the *in vivo* Zn determination was demonstrated in the laboratory using a prostate phantom in the form of a polyethylene vial filled with aqueous Zn solution. The experimental arrangement is shown in Fig. 2. The radiation source was a filtered X-ray beam **20** from a tungsten anode X-ray tube **22.** The tube **22** was operated at **36** kV and the filter **24** was a combination of Cu/Mo foils. The diameter of the beam on the sample **26** was about 10 mm. A ferrous collimator **28** was included in the beam's path. The irradiated samples consisted of 30 cc polyethylene vials, 34 mm in diameter and 1 mm thick wall, containing aqueous solutions of Zn. The Zn characteristic X-rays were emitted from the sample and detected by Si(Li) detector **30** (5 mm² in area) cooled by a liquid nitrogen (LN₂) arrangement **32.**

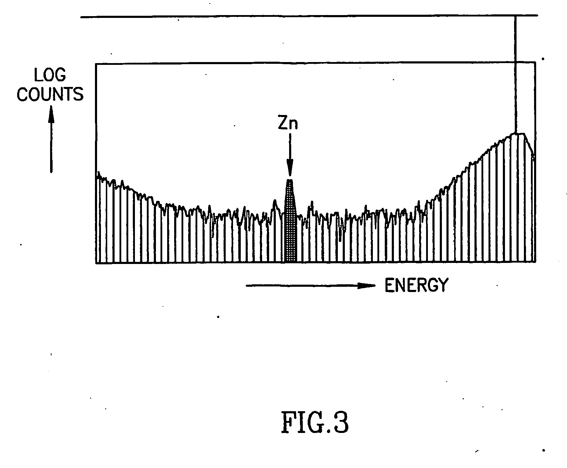

Fig. 3 shows a spectrum obtained from irradiation of a vial containing 1000 µg/g of Zn aqueous solution. As can be observed the Zn peak is very well defined and is positioned on a flat background. The small peak on the left side is due to Cu most probably present in the detector structure or housing. From this preliminary study it can be concluded that the optimal source X-ray energy for detection of Zn is in the range of 15-20 keV.

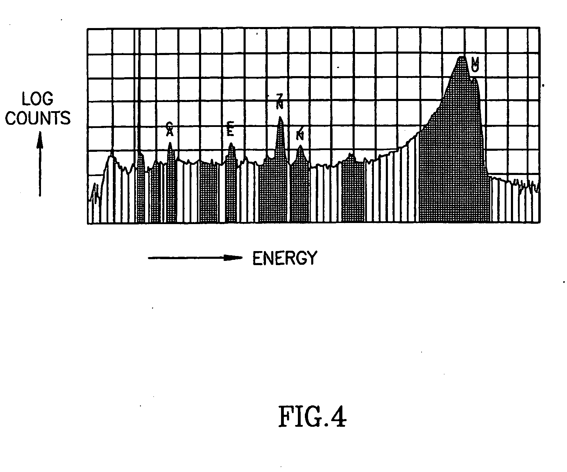

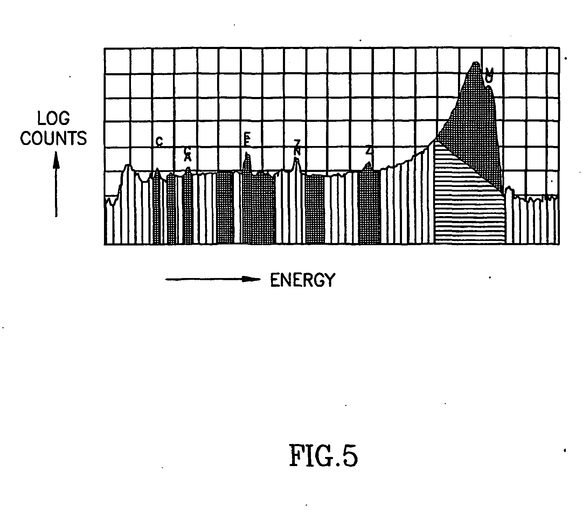

The complexity of the detected X-ray spectrum was tested *in vitro* using the XRF method on prostate samples and indicated that Zn in prostate can be accurately measured without interference from other elements. The samples were exposed to X-rays emitted from an X-ray tube with a Mo anode. The tube operated at 27 keV and the characteristic Mo X-ray line of 17.44 keV was filtered out using a crystal monochromator. The characteristic radiation emitted from the sample was measured using a Si(Li) detector having energy resolution of 160 eV at 6.4 keV. Fig. 4 and Fig. 5 show the XRF spectra obtained from prostate specimens embedded in paraffin, prepared for histological examination. As can be observed from the spectra, the Zn concentration in the cancerous tissue is much smaller than in the prostate with adenoma. Other elements such as Ca and Fe are also measurable and can be used for normalization purposes. For example, the ratio of Zn/Fe in the tissue was about 7 times lower in the case of prostate cancer.

The X-ray detector **6** can be a high energy-resolution detector such as for example cooled Si, Si(Li), HgI₂, CdZnTe or other detectors sensitive to X-rays of 8-10 keV or an array of such detectors. The detector is designed to measure the characteristic Zn X-ray lines (8.6 and 9.6 keV) emitted from the examined tissue under the X-ray irradiation. Care must be taken to avoid Cu or brass in the construction of the probe, because of the presence of Zn in brass and the proximity of the Cu characteristic lines (8.04 and 8.904 keV) to that of Zn.

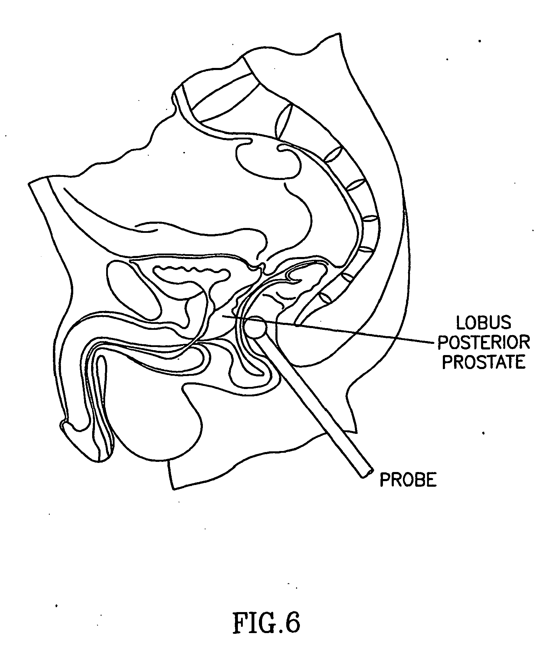

Fig. 6 depicts schematically the measurement procedure. The measurement will be performed by introducing the probe through the rectum in close proximity to the peripheral zone of the prostate. By irradiating a small area of the prostate it is possible to determine the distribution of Zn in the peripheral zone, simply by moving the probe to different positions. This is of importance for staging of the prostate cancer. Use of an array of detectors or a position-sensitive detector, will eliminate the need for scanning and will provide zinc concentration mapping in a single measurement

The elemental (e.g. Zn) mapping is important also for cancer therapy, since it can guide the treatment into the cancerous regions. Thus the recently proposed PDT can benefit from the information about the tumor localization and dimensions.

A combination of the proposed transrectal or transurethral probe with other mapping methods such as TRUS could provide an improved accuracy to biopsy procedure. Current ultrasound guided biopsy is unsatisfactory. Due to a low reliability of TRUS image, repeated biopsies are needed, with the risk of infections and extra costs.

It is estimated that a radiation dose of about 5 mSv to the peripheral tissue of the prostate will be required in order to detect Zn concentration with a statistical precision of about 5%.

## Claims

1. An apparatus for *in vivo* detection of a chemical element (7) in the prostate of a subject, comprising:
(a) a probe (1) adapted for being inserted into at least one of the rectum or the urethra of the subject, said probe comprising:
(i) a radiation source (3) being capable of exciting the chemical element to emit fluorescent x-ray radiation to form emitted radiation (5);
(ii) a radiation detector (6) capable of detecting said emitted radiation and being suitable for mapping said emitted radiation; and
(iii) a radiation-collimation system (28);
and the apparatus further comprising:
(b) means for mapping the concentration of the chemical element in the prostate, based on said mapping of said emitted radiation.

2. The apparatus of claim 1, wherein said radiation detector is selected from the group consisting of a scanning detector, a position-sensitive detector, an array of detectors sensitive to X-rays and a high energy-resolution solid state detector.

3. The apparatus of claim 2, wherein said solid state detector comprises one of a detector based on Silicon (Si), Silicon-Lithium-drifted (Si(Li)), Mercury Iodide (HgI₂) or Cadmium-Zinc Telluride (CdZnTe).

4. The apparatus of any of claims 1-3, wherein said radiation source comprises a scanning radiation source.

5. The apparatus of any of claims 1-4, wherein said means for mapping the concentration of the chemical element in the prostate provide outline of possible cancerous region in the prostate.

6. The apparatus of claim 5, wherein said means for mapping the concentration of the chemical element in the prostate provide said outline according to a distribution of the chemical element in at least a region of the prostate being examined.

7. The apparatus of claim 6, wherein said boundary is at least partially determined according to a distribution of different concentrations of the chemical element within at least said region.

8. The apparatus of claim 7, wherein said distribution of said different concentrations of the chemical element is also used for staging the cancer.

9. The apparatus of any of claims 1-8, further comprising additional mapping means for combining with information from said means for mapping the concentration of the chemical element in the prostate, for determining said boundary.

10. The apparatus of claim 9, wherein said additional mapping means comprises TRUS (transrectal ultrasound probe).

11. The apparatus of any of claims 1-10, wherein the chemical element comprises zinc, wherein said radiation detector and said radiation source are suitable for measuring the concentration of zinc, and wherein said means for mapping the concentration of the chemical element in the prostate maps the concentration of zinc to detect a possible cancer in at least a portion of the prostate.

12. The apparatus of any of claims 1-11, wherein the chemical element comprises a chemical element introduced into the prostate for a specific medical procedure, and wherein concentration of the chemical element is mapped to perform the specific medical procedure on at least a portion of the prostate.

13. The apparatus of any of claims 1-12, further comprising means for detecting the level of the chemical element in the prostate tissue, based on said emitted radiation.

14. The apparatus of any of claims 1-13, wherein said radiation detector detects X-ray fluorescence.

15. The apparatus of any of claims 1-14, wherein the chemical element to be detected comprises one or more of Zn, Fe, Ca, Br, or Pd.

16. The apparatus of claim 15, wherein the chemical element to be detected comprises Zn.

17. The apparatus of any of claims 14-16, wherein the chemical element to be detected emits characteristic fluorescent x-rays according to an identity of the chemical element, and wherein an intensity of said characteristic fluorescent x-rays correlates to a concentration of the chemical element, such that said radiation detector is adapted to detect at least one chemical element according to said characteristic fluorescent x-rays and to measure said intensity.

18. The apparatus of any of claims 1-17, wherein said radiation source comprises at least one of a radioactive source, an X-ray tube, a synchrotron light source, an X-ray beam guide connected to an external X-ray source or a miniature plasma X-ray generator.

19. The apparatus of any of claims 1-18, wherein said probe comprises a biopsy device.

20. The apparatus of any of claims 1-19, wherein said probe comprises a device for injection of a drug.

21. The apparatus of any of claims 1-20, wherein said probe comprises a device for illumination of the tumor with light.

22. The apparatus of any of claim 13, wherein said level of the chemical element is detected by normalizing measurement of said emitted radiation according to a normalizing measurement of a reference element.

23. The apparatus of any of claims 1-22, wherein the apparatus serve for localizing cancerous areas of the prostate of the subject.

24. The apparatus of any of claims 1-23, wherein said radiation-collimation system is configured for preventing said radiation detector from directly receiving radiation emitted from said radiation source.

25. The apparatus of claim 24, wherein said radiation-collimation system causes said radiation detector to receive only radiation emitted from elements in the prostate tissue under investigation.

26. The apparatus of any of claims 1-25, wherein said radiation detector is capable of detecting radiation emitted from a radioactive chemical element introduced to the prostate of the subject.

27. The apparatus of any of claims 1-26, wherein the apparatus is operable to turn off radiation emanating from said probe.

## Patentansprüche

1. Vorrichtung für den In-vivo-Nachweis eines chemischen Elements (7) in der Prostata eines Patienten, umfassend:
(a) eine Sonde (1), die zum Einführen in wenigstens eines von beiden, Rektum oder Urethra, eines Patienten angepasst ist, wobei die Sonde umfasst:
(i) eine Strahlungsquelle (3), die in der Lage ist, das chemische Element zum Abgeben von Fluoreszenzröntgenstrahlung anzuregen, um eine abgegebene Strahlung (5) zu bilden;
(ii) einen Strahlungsdetektor (6), der in der Lage ist, die abgegebene Strahlung nachzuweisen, und dazu geeignet ist, die abgegebene Strahlung abzubilden; und
(iii) ein Strahlungskollimationssystem (28);
und wobei die Vorrichtung weiter umfasst:
(b) Mittel zum Abbilden der Konzentration des chemischen Elements in der Prostata auf der Basis des Abbildens der abgegebenen Strahlung.

2. Vorrichtung nach Anspruch 1, wobei der Strahlendetektor ausgewählt ist aus der Gruppe, bestehend aus einem Abtastdetektor, einem positionsempfindlichen Detektor, einer Anordnung von röntgenstrahlempfindlichen Detektoren, und einem Festkörperdetektor mit Hochenergieauflösung.

3. Vorrichtung nach Anspruch 2, wobei der Festkörperdetektor einen Detektor umfasst, der ein Detektor auf der Basis von Silicium (Si), Silicium-Lithiumgedriftet (Si(Li)), Quecksilberiodid (Hgl₂) oder Cadmium-Zink-Tellurid (CdZnTe) ist.

4. Vorrichtung nach einem der Ansprüche 1-3, wobei die Strahlungsquelle eine Abtaststrahlungsquelle umfasst.

5. Vorrichtung nach einem der Ansprüche 1-4, wobei das Mittel zum Abbilden der Konzentration des chemischen Elements in der Prostata die Kontur eines möglichen kanzerösen Bereichs in der Prostata bereitstellt.

6. Vorrichtung nach Anspruch 5, wobei das Mittel zum Abbilden der Konzentration des chemischen Elements in der Prostata die Kontur gemäß einer Verteilung des chemischen Elements in wenigstens einem Bereich der untersuchten Prostata bereitstellt.

7. Vorrichtung nach Anspruch 7, wobei die Abgrenzung wenigstens teilweise gemäß einer Verteilung von verschiedenen Konzentrationen des chemischen Elements innerhalb wenigstens dieses Bereichs bestimmt wird.

8. Vorrichtung nach Anspruch 7, wobei die Verteilung der verschiedenen Konzentrationen des chemischen Elements auch zur Stadiumsbestimmung des Karzinoms verwendet wird.

9. Vorrichtung nach einem der Ansprüche 1-8, weiterhin umfassend zusätzliche Abbildungsmittel zum Kombinieren mit Informationen von dem Mittel zum Abbilden der Konzentration des chemischen Elements in der Prostata, um die Abgrenzung zu bestimmen.

10. Vorrichtung nach Anspruch 9, wobei das zusätzliche Abbildungsmittel eine TRUS (Transrektale Ultraschallsonde) umfasst.

11. Vorrichtung nach einem der Ansprüche 1-10, wobei das chemische Element Zink umfasst, wobei der Strahlungsdetektor und die Strahlungsquelle dazu geeignet sind, die Konzentration von Zink zu messen, und wobei das Mittel zum Abbilden der Konzentration des chemischen Elements in der Prostata die Konzentration von Zink abbildet, um ein mögliches Karzinom in wenigstens einem Teilbereich der Prostata nachzuweisen.

12. Vorrichtung nach einem der Ansprüche 1-11, wobei das chemische Element ein chemisches Element umfasst, das für eine spezifische medizinische Maßnahme in die Prostata eingebracht wird, und wobei die Konzentration des chemischen Elements abgebildet wird, um die spezifische medizinische Maßnahme an wenigstens einem Teilbereich der Prostata durchzuführen.

13. Vorrichtung nach einem der Ansprüche 1-12, weiter umfassend Mittel zum Nachweis des Gehalts an dem chemischen Element im Prostatagewebe auf der Basis der abgegebenen Strahlung.

14. Vorrichtung nach einem der Ansprüche 1-13, wobei der Strahlungsdetektor Röntgenstrahlfluoreszenz nachweist.

15. Vorrichtung nach einem der Ansprüche 1-14, wobei das nachzuweisende chemische Element eines oder mehrere von Zn, Fe, Ca, Br oder Pd umfasst.

16. Vorrichtung nach Anspruch 15, wobei das nachzuweisende chemische Element Zn umfasst.

17. Vorrichtung nach einem der Ansprüche 14-16, wobei das nachzuweisende chemische Element charakteristische Fluoreszenzröntgenstrahlen gemäß einer Identität des chemischen Elements abgibt, und wobei eine Intensität der charakteristischen Fuoreszenzröntgenstrahlen mit einer Konzentration des chemischen Elements korreliert, wobei der Strahlungsdetektor derart angepasst ist, dass er wenigstens ein chemisches Element gemäß den charakteristischen Fluoreszenzröntgenstrahlen nachweist und die Intensität misst.

18. Vorrichtung nach einem der Ansprüche 1-17, wobei die Strahlungsquelle mindestens eines von Folgendem umfasst: eine radioaktive Quelle, eine Röntgenröhre, eine synchrotrone Lichtquelle, eine mit einer externen Röntgenstrahlquelle verbundene Röntgenstrahlführung oder einen Miniaturplasmaröntgengenerator.

19. Vorrichtung nach einem der Ansprüche 1-18, wobei die Sonde eine Biopsievorrichtung umfasst.

20. Vorrichtung nach einem der Ansprüche 1-19, wobei die Sonde eine Vorrichtung zum Injizieren eines Arzneimittels umfasst.

21. Vorrichtung nach einem der Ansprüche 1-20, wobei die Sonde eine Vorrichtung zur Beleuchtung des Tumors mit Licht umfasst.

22. Vorrichtung nach Anspruch 13, wobei der Gehalt an dem chemischen Element durch normalisierende Messung der abgegebenen Strahlung gemäß einer normalisierenden Messung eines Referenzelements nachgewiesen wird.

23. Vorrichtung nach einem der Ansprüche 1-22, wobei die Vorrichtung zum Lokalisieren von kanzerösen Bereichen der Prostata des Patienten dient.

24. Vorrichtung nach einem der Ansprüche 1-23, wobei das Strahlungskollimationssystem derart konfiguriert ist, dass es den Strahlungsdetektor daran hindert, die von der Strahlungsquelle abgegebene Strahlung direkt zu empfangen.

25. Vorrichtung nach Anspruch 24, wobei das Strahlungskollimationssystem bewirkt, dass der Strahlungsdetektor nur Strahlung empfängt, die von Elementen in dem untersuchten Prostatagewebe abgegeben wird.

26. Vorrichtung nach einem der Ansprüche 1-25, wobei der Strahlungsdetektor in der Lage ist, Strahlung nachzuweisen, die von einem radioaktiven chemischen Element abgegeben wird, das in die Prostata des Patienten eingebracht wurde.

27. Vorrichtung nach einem der Ansprüche 1-26, wobei die Vorrichtung derart eingerichtet ist, dass sie Strahlung, die von der Sonde ausgeht, ausschaltet.

## Revendications

1. Appareil pour la détection *in vivo* d'un élément chimique (7) dans la prostate d'un sujet, comprenant :
(a) une sonde (1) apte à être introduite dans au moins l'un parmi le rectum ou l'urètre du sujet, ladite sonde comprenant :
(i) une source de radiation (3) qui est capable d'exciter l'élément chimique pour émettre une radiation de rayons X fluorescents afin de former une radiation émise (5) ;
(ii) un détecteur de radiation (6) capable de détecter ladite radiation émise et étant approprié pour cartographier ladite radiation émise ; et
(iii) un système (28) de collimation de radiation ; et l'appareil comprenant en outre :
(b) des moyens pour cartographier la concentration de l'élément chimique dans la prostate, sur la base de ladite cartographie de ladite radiation émise.

2. Appareil selon la revendication 1, dans lequel ledit détecteur de radiation est choisi dans le groupe constitué par un détecteur de balayage, un détecteur sensible à la position, un réseau de détecteurs sensibles aux rayons X et un détecteur à l'état solide à haute résolution en énergie.

3. Appareil selon la revendication 2, dans lequel ledit détecteur à l'état solide comprend l'un parmi un détecteur à base de Silicium (Si), de Silicium dopé au Lithium (Si(Li)), d'Iodure de Mercure (HgI₂) ou du Tellurure de Cadmium-Zinc (CdznTe).

4. Appareil selon l'une quelconque des revendications 1 à 3, dans lequel ladite source de radiation comprend une source de radiation de balayage.

5. Appareil selon l'une quelconque des revendications 1 à 4, dans lequel lesdits moyens pour cartographier la concentration de l'élément chimique dans la prostate fournissent un profil de région cancéreuse possible dans la prostate.

6. Appareil selon la revendication 5, dans lequel lesdits moyens pour cartographier la concentration de l'élément chimique dans la prostate fournissent ledit profil conformément à une distribution de l'élément chimique dans au moins une région de la prostate qui est examinée.

7. Appareil selon la revendication 6, dans lequel ladite limite est au moins partiellement déterminée conformément à une distribution de différentes concentrations de l'élément chimique à l'intérieur d'au moins ladite région.

8. Appareil selon la revendication 7, dans lequel ladite distribution desdites différentes concentrations de l'élément chimique est également utilisée pour la stadification le cancer.

9. Appareil selon l'une quelconque des revendications 1 à 8, comprenant en outre un moyen supplémentaire de cartographie pour une combinaison avec des informations provenant desdits moyens pour cartographier la concentration de l'élément chimique dans la prostate, pour déterminer ladite limite.

10. Appareil selon la revendication 9, dans lequel ledit moyen supplémentaire de cartographie comprend une sonde à ultrasons transrectale (TRUS).

11. Appareil selon l'une quelconque des revendications 1 à 10, dans lequel l'élément chimique comprend le zinc, dans lequel ledit détecteur de radiation et ladite source de radiation sont appropriés pour mesurer la concentration de zinc, et dans lequel lesdits moyens pour cartographier la concentration de l'élément chimique dans la prostate cartographient la concentration de zinc pour détecter un cancer possible dans au moins une partie de la prostate.

12. Appareil selon l'une quelconque des revendications 1 à 11, dans lequel l'élément chimique comprend un élément chimique introduit dans la prostate pour une intervention médicale spécifique, et dans lequel une concentration de l'élément chimique est cartographiée pour effectuer l'intervention médicale spécifique sur au moins une partie de la prostate.

13. Appareil selon l'une quelconque des revendications 1 à 12, comprenant en outre des moyens pour détecter le taux de l'élément chimique dans le tissu de la prostate, sur la base de ladite radiation émise.

14. Appareil selon l'une quelconque des revendications 1 à 13, dans lequel ledit détecteur de radiation détecte la fluorescence X.

15. Appareil selon l'une quelconque des revendications 1 à 14, dans lequel l'élément chimique devant être détecté comprend un ou plusieurs parmi Zn, Fe, Ca, Br ou Pd.

16. Appareil selon la revendication 15, dans lequel l'élément chimique devant être détecté comprend Zn.

17. Appareil selon l'une quelconque des revendications 14 à 16, dans lequel l'élément chimique devant être détecté émet des rayons X fluorescents caractéristiques conformément à une identité de l'élément chimique, et dans lequel une intensité desdits rayons X fluorescents caractéristiques est corrélée à une concentration de l'élément chimique, de telle sorte que ledit détecteur de radiation est adapté pour détecter au moins un élément chimique conformément auxdits rayons X fluorescents caractéristiques et pour mesurer ladite intensité.

18. Appareil selon l'une quelconque des revendications 1 à 17, dans lequel ladite source de radiation comprend au moins l'un parmi une source radioactive, un tube à rayons X, une source de lumière de synchrotron, un guide de faisceau de rayons X connecté à une source de rayons X externe ou un générateur de rayons X à plasma miniature.

19. Appareil selon l'une quelconque des revendications 1 à 18, dans lequel ladite sonde comprend un dispositif de biopsie.

20. Appareil selon l'une quelconque des revendications 1 à 19, dans lequel ladite sonde comprend un dispositif pour l'injection d'un médicament.

21. Appareil selon l'une quelconque des revendications 1 à 20, dans lequel ladite sonde comprend un dispositif pour l'illumination de la tumeur par de la lumière.

22. Appareil selon la revendication 13, dans lequel ledit taux de l'élément chimique est détecté par une mesure de normalisation de ladite radiation émise conformément à une mesure de normalisation d'un élément de référence.

23. Appareil selon l'une quelconque des revendications 1 à 22, dans lequel l'appareil sert à localiser des zones cancéreuses de la prostate du sujet.

24. Appareil selon l'une quelconque des revendications 1 à 23, dans lequel ledit système de collimation de radiation est configuré pour empêcher ledit détecteur de radiation de recevoir directement une radiation émise par ladite source de radiation.

25. Appareil selon la revendication 24, dans lequel ledit système de collimation de radiation amène ledit détecteur de radiation à recevoir seulement une radiation émise par des éléments dans le tissu de la prostate soumis à la recherche.

26. Appareil selon l'une quelconque des revendications 1 à 25, dans lequel ledit détecteur de radiation est capable de détecter une radiation émise par un élément chimique radioactif introduit dans la prostate du sujet.

27. Appareil selon l'une quelconque des revendications 1 à 26, dans lequel l'appareil est actionnable pour couper une radiation émanant de ladite sonde.
